# EUROPEAN PATENT APPLICATION

(11) **EP 4 603 022 A1**
(43) Date of publication of application: **20.08.2025**
(21) Application number: 24857688.6
(22) Date of filing: 07.03.2024
(51) Int. Cl.: A61B 5/332

(54) **PORTABLE ELECTROCARDIOGRAM MONITOR**

(30) Priority: 30.08.2023 CN 202311105781; 30.08.2023 CN 202311105756; 31.08.2023 CN 202311109396
(71) Applicant: Beijing Weheal Technology Co., Ltd., Beijing 102308 (CN); Qingdao Weheal Technology Co., Ltd., Qingdao, Shandong 266308 (CN)
(72) Inventor: YU, Haipeng, Beijing 102308 (CN)
(74) Representative: Huang, Liwei
(86) International application number: PCT/CN2024/080512
(87) International publication number: WO 2025/044135

(57) **Abstract**

A portable electrocardiogram monitor, comprising an electrocardiogram electrode holder (2) and an electrocardiogram monitor main unit (3), wherein the electrocardiogram electrode holder (2) comprises a holder body (21) and holder arms including an upper holder arm (22), a lower holder arm (23) and a side holder arm (24), the upper holder arm (22), the lower holder arm (23) and the side holder arm (24) all being turnable by a target angle toward the proximal face of the holder body (21) around their respective connection positions with the holder body (21); and the electrocardiogram monitor main unit (3) is detachably mounted on the holder body (21). The portable electrocardiogram monitor can solve the technical problems of the electrocardiogram devices in the prior art having a non-compact structure and poor portability and being inconvenient for application due to inconvenient positioning of electrocardiogram collection positions, and avoids the entanglement of long and numerous cables in the prior art. The holder arms can all be connected to the corresponding edges of the holder body (21) in a turnable manner, such that when a user to be monitored is required to perform electrocardiogram monitoring, positioning can be easily achieved by means of unfolding the holder arms without requiring professional operation, and the user to be monitored can perform positioning and complete the electrocardiogram monitoring only based on the reference positioning datum on the holder body (21), such that the operation is simple and easy to implement.

## Description

### Cross-Reference to Related Applications

The present disclosure is based on and claims priority to CN Application No. 202311105781.6 filed on August 30, 2023, CN Application No. 202311105756.8 filed on August 30, 2023 and CN Application No. 202311109396.9 filed on August 31, 2023, the disclosures of which are hereby incorporated by reference herein in their entireties.

### Technical Field of the Invention

The present invention belongs to the technical field of portable electrocardiogram monitor design, and particularly relates to a portable electrocardiogram monitor.

### Background of the Invention

As an important means to detect cardiovascular diseases, the electrocardiography acquires bioelectrical signals through electrodes (also referred to as detection electrodes) covering the human body surface, so as to record the activity rhythm of the heart. The 12-lead electrocardiogram monitors with clinically diagnostic significance mostly adopt the Wilson's 12-lead system. Since there are numerous leads, these leads are easy to tangle up and the detection points are complex, the electrocardiogram (ECG) detection systems with this structure need to be operated by professional, and thus cannot be conveniently used in the general population.

Based on the above defects of the conventional 12-lead electrocardiogram monitors, the applicant previously proposed portable 12-lead electrocardiogram monitors. However, most of these electrocardiogram monitors are not equipped with corresponding matching display components, so the ECG related parameters need to be displayed by means of intelligent terminals such as mobile phones and tablet computers, and data acquisition and analysis need to be performed through network or Bluetooth connection. As a result, these electrocardiogram monitors are limited to a certain extent and easily restricted by the terminal equipment or network. In addition, since the electrocardiogram monitors are considerably used by elderly people and the elderly people have certain obstacles in using smart phones, it is difficult for these users to view ECG parameters by using intelligent terminals, thus reducing the application and popularization of the electrocardiogram monitors.

Some portable ECG apparatuses have been proposed in the related art, but these portable ECG apparatuses are difficult to adapt to individual differences among different users' body shapes. Particularly, during specific detection, the V6 detection electrode needs to be accurately positioned at the left 3/4 intercostal midaxillary line of the human body, but the support arm with fixed length cannot adapt to different body shapes, especially users who greatly differ in fatness. Especially, for a support arm that is designed to be turned for retraction, since the position of the turning connection is relatively determined, the position of the V6 detection electrode cannot be adjusted for users with different body shapes by changing the relative position between the support arm and the support main body. Consequently, since the detection electrode contact is not accurately positioned at the target position during ECG detection, the output results of ECG detection are possibly distorted.

Thus, it is an urgent technical problem for those skilled in the art to provide an improved portable electrocardiogram monitor that is compact in structure and convenient to carry, locate and use, based on the defects in the prior art.

### Summary of the Invention

In a first aspect, the present invention provides a portable electrocardiogram monitor, which can solve the technical problems of incompact structure, poor portability and inconvenient use due to inconvenient ECG acquisition position of the ECG detection apparatus in the prior art.

In another aspect, the portable electrocardiogram monitor provided by the present invention can solve the technical problem in the prior art that the ECG parameters need to be displayed by means of intelligent terminals such as mobile phones and tablet computers, it is easily restricted by the terminal equipment or network and it is inconvenient for application and popularization in the elderly population.

In still another aspect, the portable electrocardiogram monitor provided by the present invention can solve the technical problem in the prior art that the V6 detection electrode on the support arm cannot be accurately positioned at target positions on different body shapes and the output results of ECG detection are possibly distorted since the length of the ECG detection electrode support arm connected to the support main body in a turning manner cannot be adjusted.

To solve the problem in the first aspect, the present invention provides a portable electrocardiogram monitor, including an ECG detection electrode support and an electrocardiogram monitor host;
wherein the ECG detection electrode support includes a support main body and support arms, and the support arms include an upper support arm, a lower support arm and a side support arm;
the upper support arm, the lower support arm and the side support arm each can be turned by a target angle toward a proximal surface of the support main body around its respective connection with the support main body; and
the electrocardiogram monitor host is detachably mounted on the support main body.

In some implementations,
an LA detection electrode and an RA detection electrode are provided on a distal surface of the support main body, and a V3 detection electrode and an RL detection electrode are provided on the proximal surface thereof;
the upper support arm is connected to an upper edge of the support main body, and a V1 detection electrode and a V2 detection electrode are provided on a proximal surface of the upper support arm;
the lower support arm is connected to a lower edge of the support main body, and an LL detection electrode is provided on a proximal surface of the lower support arm; and
the side support arm is connected to a left edge of the support main body to correspond to a chest side where the heart of a user to be detected is located, and a V4 detection electrode, a V5 detection electrode and a V6 detection electrode are provided on a proximal surface of the side support arm.

In some implementations,
a locking part is provided on the proximal surface of the support main body, and the side support arm has a detection position and a retraction position; when the side support arm is at the detection position, the V4 detection electrode, the V5 detection electrode and the V6 detection electrode each is located at a preset ECG detection position; and, when the side support arm is at the retraction position, the locking part locks the side support arm so that the proximal surface of the side support arm is opposite to the proximal surface of the support main body.

In some implementations,
the V4 detection electrode, the V5 detection electrode and the V6 detection electrode are arranged at intervals in a length direction of the arm body of the side support arm from a side close to the support main body to a side away from the support main body; the locking part includes a locking key; the locking key has a first locking position and a first unlocking position; a ring groove is formed on the V6 detection electrode; a first bump is provided on the locking key; when the side support arm is at the retraction position, the first bump is at least partially inserted into the ring groove to be located at the first locking position; and, when the locking key is at the first unlocking position, the first bump escapes from the ring groove.

In some implementations,
an assembling hole for allowing the electrocardiogram monitor host to be embedded therein is constructed on the support main body; the locking key also has a second locking position and a second unlocking position; a groove is formed on a sidewall of the electrocardiogram monitor host; a second bump is also provided on the locking key; when the electrocardiogram monitor host is embedded into the assembling hole, the second bump is at least partially inserted into the groove to be located at the second locking position; and, when the locking key is at the second unlocking position, the second bump escapes from the groove.

In some implementations,
the locking key is connected to a slide groove on the support main body in a sliding manner, and an elastic element is provided between the locking key and the support main body; and, the elastic element can exert an elastic force to the locking key so that the locking key can slide close to the V6 detection electrode at the retraction position and the embedded electrocardiogram monitor host.

In some implementations,
the insertion fitting depth of the first bump and the ring groove is less than the insertion fitting depth of the second bump and the groove; and/or, the locking key has a force exerting bump protruded from the proximal surface of the support main body.

In some implementations,
a third bump is provided on an edge of the proximal surface of the side support arm; and, when the side support arm, the upper support arm and the lower support arm are all at the retraction positions, the third bump is clamped with the V1 detection electrode, the V2 detection electrode and the LL detection electrode to prevent the upper support arm and the lower support arm from being turned away from the support main body.

In some implementations,
the LA detection electrode and the RA detection electrode are detection electrode sheets; and, the V1 detection electrode, the V2 detection electrode, the V3 detection electrode, the V4 detection electrode, the V5 detection electrode, the V6 detection electrode, the RL detection electrode and the LL detection electrode are all detection electrode columns.

In some implementations,
the electrocardiogram monitor host includes a host component and a display component; the electrocardiogram monitor host is assembled on the distal surface of the support main body through the host component; the host component includes a host shell and an ECG detection circuit board arranged in the host shell; the ECG detection circuit board is electrically connected to each of the detection electrodes; the display component includes a display screen assembly; and, the display screen assembly is at least configured to display ECG parameters output by the ECG detection circuit board.

In some implementations,
the display component has a display shell; the display screen assembly is located on a side face of the display shell away from the host shell; a first side of the display shell is pivotally connected to a first side of the host shell through a pivot structure; and, the display component can be driven to unfold or fold around the pivot structure relative to the host component.

In some implementations,
the assembling hole is constructed on the support main body, and the host component is embedded into the assembling hole; an electrical switching component electrically connected to the ECG detection circuit board is provided on a bottom wall of the host shell, and an electrical connection component electrically connected to each of the detection electrodes is provided on a wall of the assembling hole; and, when the host component is assembled in the assembling hole, the electrical connection component is connected to the electrical switching component.

In some implementations, the length of the side support arm is adjustable, and the side support arm includes:
a fixed arm section, one end of which is connected to the support main body of the electrocardiogram monitor in a turnable manner, a first detection electrode being provided on a proximal surface of the fixed arm section, the fixed arm section being also provided with a slide rail arranged in a length extension direction of the fixed arm section, the first detection electrode being the V4 detection electrode in some implementations; and
a telescopic arm section, on a proximal surface of which being provided with a second detection electrode, a slider being also provided on the telescopic arm section, the slider being sleeved on the outer side of the slide rail in the length direction of the slide rail in a sliding manner, the second detection electrode being the V5 detection electrode and the V6 detection electrode in some implementations.

In some implementations,
the slide rail has a wiring groove running through two ends of the slide rail in its length direction; the wiring groove has a U-shaped cross-section; the opening of the U-shape faces the side of proximal surfaces of the fixed arm section and the telescopic arm section; and, the respective cables of the first detection electrode and the second detection electrode at least partially pass through the wiring groove.

In some implementations,
each of two opposite sidewalls of the slide rail is constructed with a slideway extending in a length direction thereof, a plurality of ball holes are formed at positions on the slider corresponding to each of the slideways, and a ball is clamped between each of the ball holes and the slideway.

In some implementations,
a positioning rib is provided on a wall surface of the slide rail away from the wiring groove, and a plurality of positioning bumps are provided on the positioning rib.

In some implementations,
a limiting hole is formed at an open end of the slideway, and a limiting member is connected in the limiting hole; and/or, positioning holes are also constructed on the two sidewalls of the slide rail on which the slideways are constructed.

In some implementations,
the fixed arm section further includes a first electrode shell, the first detection electrode is located on a proximal surface of the first electrode shell, and the first electrode shell is detachably connected to the proximal surfaces of the first fixed shell and the second fixed shell.

In some implementations,
the telescopic arm section further includes a second electrode shell, and the second detection electrode is located on a proximal surface of the second electrode shell; and/or, the electrode column casing of the first detection electrode is integrated with the first electrode shell, and the electrode column casing of the second detection electrode is integrated with the second electrode shell.

In some implementations,
a rotating shaft assembly is assembled on the fixed arm section; and the rotating shaft assembly includes a rotating shaft; the rotating shaft has a connecting portion; and, the connecting portion is used for detachable connection to the support main body.

In some implementations,
the rotating shaft assembly further includes an outer barrel; the rotating shaft is coaxially connected to the outer barrel in an axially movable manner; a spiral elastic element is sleeved on the outer side of the rotating shaft; and, one end of the spiral elastic element is fixedly connected to the outer barrel, while the other end thereof is fixedly connected to the rotating shaft.

The portable electrocardiogram monitor provided by the present invention has the following beneficial effects.

Ten detection electrodes are reasonably arranged on the support main body, the upper support arm, the lower support arm and the side support arm according to the ECG detection position, and the corresponding electrode leads are arranged inside the support, so that the structure is compact, and the tangling problem due to numerous long cables in the prior art is avoided. Each support arm can be connected to the respective edge of the support main body in a turning manner. When it is necessary to detect an ECG of a user to be detected, it can be easy to unfold and position each support arm, and the user to be detected can complete positioning and ECG detection according to only the positioning reference on the support main body without professional operation, so that it is easy to operate and detect, it is advantageous to promote the progress of ECG entering families, and it is possible for patients to detect ECG at home. When it is unnecessary to perform ECG detection, each support arm is turned and folded toward the side close to the proximal surface of the support main body to realize quick retraction of each support arm, so that the electrocardiogram monitor in the retracted state is compact in structure. It should be particularly noted that the detection surface of each detection electrode on each support arm in the turned and folded state faces the proximal surface of the support main body and is hidden inward as a whole, so that the detection electrodes can be protected, and the damage probability of each detection electrode due to wear or fall can be effectively reduced.

The side support arm is locked through the concave-convex matching structure of the first bump on the locking key and the ring groove on the V6 detection electrode, so that the structure is simple, and the locking operation is convenient.

The locking key can lock both the side support arm and the electrocardiogram monitor host, so that the structural design of the electrocardiogram monitor is simplified, and the whole structure of the ECG detection electrode support can be more compact.

The locking key has two distances for sliding away. The sliding in the shorter distance realizes the unlocking of the side support arm, and ECG detection can thus be carried out. The sliding in the longer distance realizes the unlocking of the electrocardiogram monitor host, and the host can be replaced in this case.

In some implementations, the electrocardiogram monitor host is integrally arranged on the detection electrode support, and the display screen assembly of the electrocardiogram monitor host can directly display the ECG parameters output by the ECG detection circuit board without the help of intelligent terminals such as mobile phones and tablet computers. Therefore, the electrocardiogram monitor is not restricted by the terminal equipment or network and is especially suitable for the elderly population, and thus is easy for application and popularization in the elderly population.

In some implementations, on one hand, the first end of the ECG detection electrode support arm is connected to the support main body in a turnable manner, so that the proximal surface of the support arm can be opposite to the proximal surface of the support main body after being turned, thereby folding and retracting the support arm to improve the structure compactness, at the same time physically protecting each detection electrode on the support arm and avoiding the damage to the detection electrode. On the other hand, on the basis that the support arm can be turned, the support arm is designed as a telescopic structure, so that the second detection electrode on the support arm can be adjusted according to different body shapes of users and accurately positioned at target positions for different body shapes, thereby improving the accuracy of the output results of ECG detection of the electrocardiogram monitor.

### Brief Description of the Drawings

To describe the implementations of the present invention or the technical solutions in the prior art more clearly, the drawings to be used in the description of the implementations or the prior art will be briefly described below. Apparently, the drawings in the following description are only exemplary, and other implementation drawings can be obtained by extension according to the provided drawings by a person of ordinary skill in the art without paying any creative effort.

The structure, scale, size and the like shown in the drawings of this specification are merely used for understanding and reading by those skilled in the art in combination with the contents disclosed in this specification and are not intended to limit the implementable conditions of the present invention, and thus have no any technically substantive meaning. Without influencing the effects and objectives which can be achieved by the present invention, any structural modification, change in scale, or size adjustment shall fall into the scope covered by the technical contents disclosed by the present invention.
FIG. 1 is a schematic diagram of stereoscopic structure of the ECG detection electrode support according to an embodiment of the present invention (from the distal perspective), where each support arm is in the ECG detection state;
FIG. 2 is a schematic diagram of stereoscopic structure of the ECG detection electrode support according to an embodiment of the present invention (from the distal perspective), where each support arm is in the turned and retracted state;
FIG. 3 is a schematic diagram of a state after assembling the ECG detection electrode support and the electrocardiogram monitor host according an embodiment of the present invention;
FIG. 4 is a schematic structure diagram of FIG. 3 from the proximal perspective and in the ECG detection state;
FIG. 5 is a schematic structure diagram of FIG. 3 from the proximal perspective and in the turned and retracted state;
FIG. 6 is a schematic diagram of the relative position relationship between the locking key at the first locking position and the V6 detection electrode in the present invention;
FIG. 7 is a schematic diagram (sectional view) of the relative position relationship between the locking key at the first locking position and the V6 detection electrode in the present invention;
FIG. 8 is a schematic diagram of stereoscopic structure of the locking key in the present invention (including the elastic element);
FIG. 9 is a sectional view of the locking key in FIG. 8;
FIG. 10 is a schematic diagram of stereoscopic structure of the electrocardiogram monitor host in FIG. 3, where both the display component and the host component are in the unfolded state;
FIG. 11 is a schematic diagram of stereoscopic structure of the electrocardiogram monitor host according to an embodiment of the present invention, where both the display component and the host component are in the buckled state;
FIG. 12 is a schematic diagram of stereoscopic structure of the electrocardiogram monitor host according to an embodiment of the present invention from another perspective;
FIG. 13 is a schematic sectional view of the electrocardiogram monitor host in FIG. 3;
FIG. 14 is a schematic diagram of stereoscopic structure of the ECG detection electrode support arm with an adjustable length according to an embodiment of the present invention from one perspective;
FIG. 15 is a schematic diagram of internal structure of FIG. 14 after the fixed section panel and the telescopic section panel are hidden;
FIG. 16 is a schematic diagram of stereoscopic structure of the ECG detection electrode support arm in FIG. 14 from another perspective;
FIG. 17 is a top view of the ECG detection electrode support arm in FIG. 16;
FIG. 18 is a sectional view of FIG. 17 along A-A;
FIG. 19 is a sectional view of FIG. 17 along C-C, i.e., a sectional view of the rotating shaft assembly in Fig .14;
FIG. 20 is a schematic diagram of stereoscopic structure of the rotating shaft assembly in FIG. 14;
FIG. 21 is a schematic diagram of stereoscopic structure of the ECG detection electrode support arm in FIG. 14 from still another perspective; and
FIG. 22 is a schematic diagram of stereoscopic structure of the slide rail in FIG. 15.

The reference numerals are represented as follows:
2: ECG detection electrode support; 21: support main body;
211: LA detection electrode; 212: RA detection electrode; 213: V3 detection electrode; 214: RL detection electrode; 215: assembling hole; 2151: electrical connection component; 2152: host fixation bump;
22: upper support arm; 221: V1 detection electrode; 222: V2 detection electrode;
23: lower support arm; 231: LL detection electrode;
24: side support arm; 241: V4 detection electrode; 242: V5 detection electrode; 243: V6 detection electrode; 2431: ring groove;
246: fixed arm section; 241: V4 detection electrode (first detection electrode); 2462: slide rail; 24621: wiring groove; 24622: slideway; 24623: positioning rib; 246231: positioning bump; 24624: limiting hole; 24625: positioning hole; 24631: first fixed shell; 246311: flanged ring; 24632: second fixed shell; 2464: first electrode shell; 2465: rotating shaft assembly; 24651: rotating shaft; 246511: connecting portion; 24652: outer barrel; 24653: spiral elastic element; 24654: gasket; 24655: screw; 2466: fixed section panel;
247: telescopic arm section; 2473: slider; 2474: second electrode shell; 2475: telescopic section panel;
25: locking key;
251: first bump; 252: second bump; 253: force exerting bump; 254: elastic element;
3: electrocardiogram monitor host;
31: host component; 311: host shell; 312: ECG detection circuit board;
32: display component; 321: display shell; 322: display screen assembly; 325: display control circuit board;
34: electrical switching component; 35: power supply battery; 36: power switch; 37: data export interface; 38: charging interface.

### Detailed Description of the Invention

The technical solutions in the embodiments of the present invention will be described below clearly and completely with reference to the drawings in the embodiments of the present invention. Apparently, the embodiments described herein are only some but not all of the embodiments of the present invention. The following description of at least one exemplary embodiment is merely illustrative in nature and is not regarded as any limitations to the present invention and application or use thereof. All other embodiments obtained by a person of ordinary skill in the art without paying any creative effort on the basis of the embodiments in the present invention shall fall into the protection scope of the present invention.

It is to be noted that the terms used herein are merely used for describing specific implementations, rather than limiting exemplary implementations of the present invention. As used herein, unless otherwise specified in the context, a singular form also includes a plural form. In addition, it should be understood that the term "include" and/or "comprise", when used in this specification, means the presence of features, steps, operations, devices, assemblies and/or combinations thereof.

It should be understood that, as used herein, the term "and/or" is merely an association relation describing associated objects, and means that there may be three relations. For example, A and/or B may refer to the following three situations: there exists A alone; there exist both A and B; and, there exists B alone. In addition, the character "/" used herein generally indicates that there is an "or" relationship between associated objects.

Unless otherwise specifically indicated, the relative arrangements, numerical expressions and numerical values of the components and steps described in these embodiments are not intended to limit the scope of the present invention. Moreover, it should be understood that, for the convenience of description, the size of each part shown in the drawings is not drawn according to the actual scale relationship. The technologies, methods and devices known to a person of ordinary skill in the related art may not be discussed in detail, but if appropriate, the discussed technologies, methods and devices should be regarded as part of the authorization specification. In all examples shown and discussed herein, any specific values should be interpreted as being merely exemplary, but not as limitations. Therefore, other examples of the exemplary embodiments may have different values. It is to be noted that, similar reference numerals and letters indicate similar items in the following drawings. Therefore, once a certain item is defined in one drawing, it is unnecessary to further discuss this item in the subsequent drawings.

In the description of the present invention, it should be understood that the orientation or position relation indicated by "front, back, up, down, left, right", "transverse, longitudinal, vertical, horizontal", "top, bottom" or other location nouns is generally based on the orientation or position relation shown in the drawings, and is merely for describing the present invention and simplifying description. Unless otherwise stated, these location nouns do not indicate or imply that the indicated apparatus or element must have a particular orientation or be constructed and operated in a particular orientation, and thus cannot be interpreted as limitations to the protection scope of the present invention. In addition, the location nouns "inside and outside" refer to the inside and outside relative to the contour of each component itself.

For the convenience of description, spatial relative terms such as "on", "above", "on the upper surface of" and "upper" can be used herein for describing the spatial position relation between one device or feature and other devices or features as shown in the drawings. It should be understood that the spatial relative terms are intended to encompass different orientations in use or operation in addition to the orientation of the device depicted in the drawings. For example, if the device in the drawing is placed upside down, the device described as "above another device or construction" or "on another device or construction" will be positioned as "below another device or construction" or "under another device or construction". Therefore, the exemplary term "above" may include two orientations, i.e., "above" and "below". This device may also be positioned in other different orientations (rotated by 90 degrees or in other orientations), and the spatial relative description used here is explained accordingly.

In addition, it is to be noted that the words such as "first" and "second" are used to define parts only for the convenience of distinguishing the corresponding parts. Unless otherwise indicated, the above words have no special meaning and thus cannot be interpreted as limitations to the protection scope of the present invention.

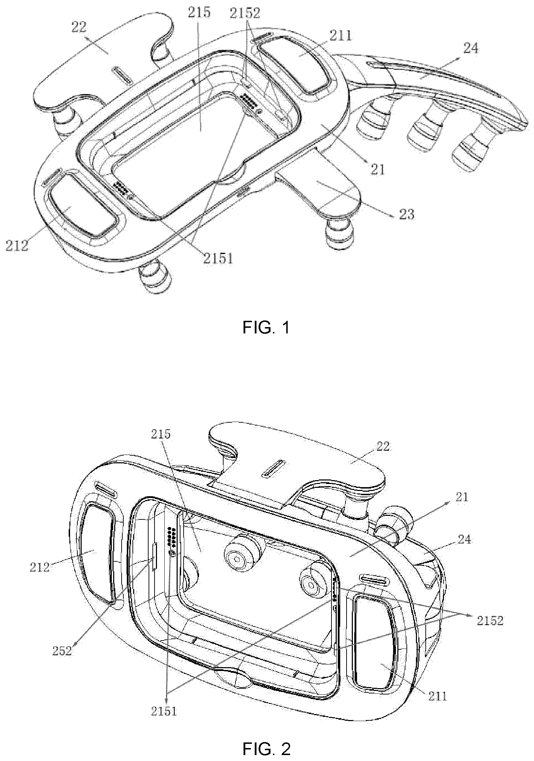

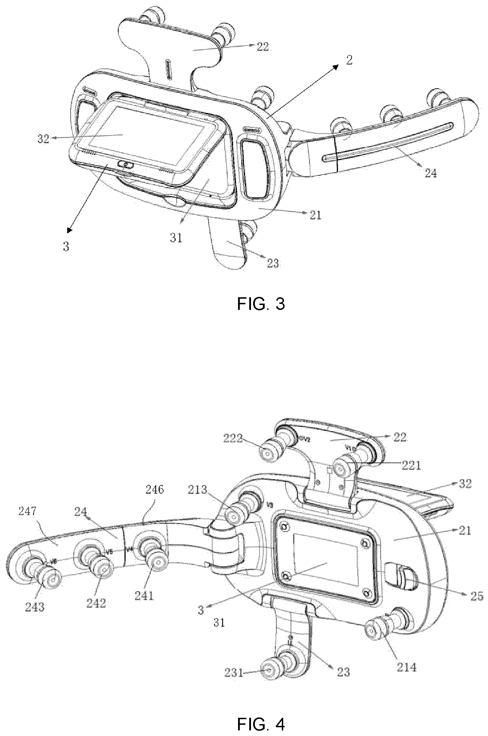

As shown in FIGS. 1-9, according to an embodiment of the present invention, an ECG detection electrode support is provided, including: a support main body 21, on a distal surface of which being provided with an LA detection electrode 211 and an RA detection electrode 212 separately corresponding to a left arm and a right arm of a user to be detected, and on a proximal surface of which being provided with a V3 detection electrode 213 and an RL detection electrode 214; an upper support arm 22, which is connected to an upper edge of the support main body 21, a V1 detection electrode 221 and a V2 detection electrode 222 being provided on a proximal surface of the upper support arm 22; a lower support arm 23, which is connected to a lower edge of the support main body 21, an LL detection electrode 231 being provided on a proximal surface of the lower support arm 23; and, a side support arm 24, which is connected to a left edge of the support main body 21 to correspond to a chest side where the heart of the user to be detected is located, a V4 detection electrode 241, a V5 detection electrode 242 and a V6 detection electrode 243 being provided on a proximal surface of the side support arm 24. The upper support arm 22, the lower support arm 23 and the side support arm 24 each can be turned by a target angle toward the proximal surface of the support main body 21 around its respective connection with the support main body 21. The aforementioned upper edge, the lower edge and the left edge are determined according to the orientation of the electrocardiogram monitor of the present invention in the use state relative to the user to be detected. The aforementioned target angle is limited to the maximum angle at which the upper support arm 22, the lower support arm 23 and the side support arm 24 can be turned close to the proximal surface of the support main body 21, so that each support arm is retracted more compactly. In a specific embodiment, the target angle at which the upper support arm 22 and the lower support arm 23 can be turned should not be less than 90° , and the target angle at which the side support arm 24 can be turned should not be less than 120° . The ten detection electrodes, i.e., the LA detection electrode 211, the RA detection electrode 212, the V3 detection electrode 213, the RL detection electrode 214, the V1 detection electrode 221, the V2 detection electrode 222, the LL detection electrode 231, the V4 detection electrode 241, the V5 detection electrode 242 and the V6 detection electrode 243, are named according to the same rules as the related detection electrodes (columns or sheets) of the conventional 12-lead ECG detector, and the arrangement position of each detection electrode is designed according to the ergonomics and meets the requirements of the known 12-lead Wilson positioning system, which is not specifically limited in the present invention.

In this technical solution, the ten detection electrodes are reasonably arranged on the support main body 21, the upper support arm 22, the lower support arm 23 and the side support arm 24 according to the ECG detection position, and the corresponding electrode leads are arranged inside the support, so that the structure is compact, and the tangling problem due to numerous long cables in the prior art is avoided. Each support arm can be connected to the respective edge of the support main body 21 in a turning manner. When it is necessary to detect an ECG of a user to be detected, each support arm can be easily unfolded and positioned, and the user to be detected can position and complete ECG detection according to only the positioning reference on the support main body 21 without professional operation, so that it is easy to operate and detect, it is advantageous to promote the progress of ECG entering families, and it is possible for patients to detect ECG at home. When it is unnecessary to perform ECG detection, each support arm is turned and folded toward the side close to the proximal surface of the support main body 21 to realize quick retraction of each support arm, so that the electrocardiogram monitor in the retracted state is compact in structure. It should be particularly noted that the detection surface of each detection electrode on each support arm retracted in the turned and folded state will face the proximal surface of the support main body 21 and is hidden inward as a whole, so that the detection electrodes can be protected, and the damage probability of each detection electrode due to wear or fall can be effectively reduced.

The aforementioned proximal surface refers to the sidewall of the electrocardiogram monitor close to the user to be detected in the use state, and the distal surface refers to the sidewall away from the user to be detected in this state. The proximal surface and the distal surface are opposite to each other.

Specifically, the aforementioned upper support arm 22, lower support arm 23 and side support arm 24 can be connected to the support main body 21 in a turnable manner through existing torsion rotating shaft structures (not shown and not marked). When the electrocardiogram monitor is in the ECG detection state, each torsion rotating shaft structure can exert a force to the respective support arm so that each support arm has a tendency of turning toward the turned and retracted state.

To ensure the position of each detection electrode, the aforementioned side support arm 24 is designed to be relatively long, while the upper support arm 22 and the lower support arm 23 are designed to be relatively short.

In some implementations,
a locking part (not marked in the drawings) is provided on the proximal surface of the support main body 21, and the side support arm 24 has a detection position (corresponding to the ECG detection state of the electrocardiogram monitor) and a retraction position (corresponding to the turned and retracted state of the electrocardiogram monitor); when the side support arm 24 is at the detection position, the V4 detection electrode 241, the V5 detection electrode 242 and the V6 detection electrode 243 each are located at a preset ECG detection position; and, when the side support arm 24 is at the retraction position, the locking part locks the side support arm 24 so that the proximal surface of the side support arm 24 is opposite to the proximal surface of the support main body 21.

Since three detection electrodes are arranged on the side support arm 24 and the side support arm 24 is relatively long, the mass of the side support arm 24 itself is relatively large, and the torsion effect of the torsion rotating shaft structure between the side support arm 24 and the support main body 21 will be weakened after long-time use. As a result, the side support arm 24 cannot be reliably and stably located at the aforementioned retraction position, and the loose state will reduce the user's satisfaction. More importantly, the corresponding detection electrode is prone to randomly collide with an external object due to the unreliable retraction position, thereby damaging the detection electrode. However, in this technical solution, by providing the locking part on the proximal surface of the support main body 21, it is ensured that the side support arm 24 can be reliably and stably located at the retraction position, thereby effectively avoiding the damage caused by the direct collision of each detection electrode on the side support arm 24 with the external object.

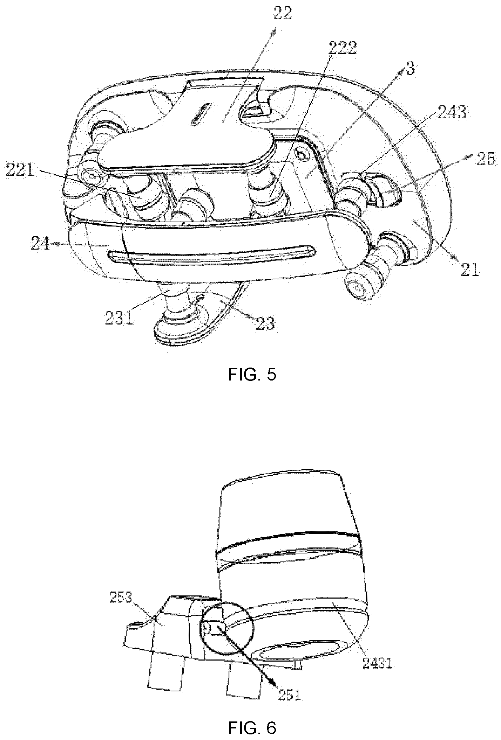

As shown in FIGS. 6-9, the V4 detection electrode 241, the V5 detection electrode 242 and the V6 detection electrode 243 are arranged at intervals from a side close to the support main body 21 to a side away from the support main body 21 in a length direction of the arm body of the side support arm 24. The locking part includes a locking key 25. The locking key 25 has a first locking position and a first unlocking position. A ring groove 2431 is formed on the V6 detection electrode 243, and a first bump 251 is provided on the locking key 25. When the side support arm 24 is at the retraction position, the first bump 251 is at least partially inserted into the ring groove 2431 to be located at the first locking position; and, when the locking key 25 is at the first unlocking position, the first bump 251 escapes from the ring groove 2431.

In this technical solution, the side support arm 24 is locked through the concave-convex matching structure of the first bump 251 on the locking key 25 and the ring groove 2431 on the V6 detection electrode 243, so that the structure is simple, and the locking operation is convenient.

As shown in FIGS. 1 and 2, in some implementations, an assembling hole 215 for allowing the electrocardiogram monitor host 3 to be embedded therein is constructed on the support main body 21. The assembling hole 215 is roughly located in a central region of the support main body 21. The locking key 25 also has a second locking position and a second unlocking position. A groove (not shown and not marked in drawings) is formed on a sidewall of the electrocardiogram monitor host 3, and a second bump 252 is also provided on the locking key 25. When the electrocardiogram monitor host 3 is embedded into the assembling hole 215, the second bump 252 is at least partially inserted into the groove to be located at the second locking position; and, when the locking key 25 is at the second unlocking position, the second bump 252 escapes from the groove. The electrocardiogram monitor host 3 is embedded into the assembling hole 215, and is in an electrical connection (e.g., communication connection) to each detection electrode through an electrical connection component 2151. The embedding structure is a detachable structure, so that a different type of the electrocardiogram monitor host 3 can be configured according to the user's actual needs. For example, a host with the display component 32 or a host without the display component can be selectively configured. The aforementioned electrical connection component 2151 can be specifically a commercially-available pogopin interface.

In this technical solution, the locking key 25 also locks the electrocardiogram monitor host 3 through the second bump 252, so that the electrocardiogram monitor host 3 can be reliably and stably embedded into the assembling hole 215. Thus, the locking key 25 in the present invention can achieve the purpose of locking both the side support arm 24 and the electrocardiogram monitor host 3, so that the structural design of the electrocardiogram monitor is simplified, and the whole structure of the ECG detection electrode support can be more compact.

It should be understood that, a host fixation bump 2152 is also provided on the inner wall of the assembling hole 215 on the side opposite to the second bump 252, and the host fixation bump 2152 can be inserted into the corresponding clamping slot on the electrocardiogram monitor host 3, so that the reliability of the position of the electrocardiogram monitor host 3 in the assembling hole 215 can be further improved.

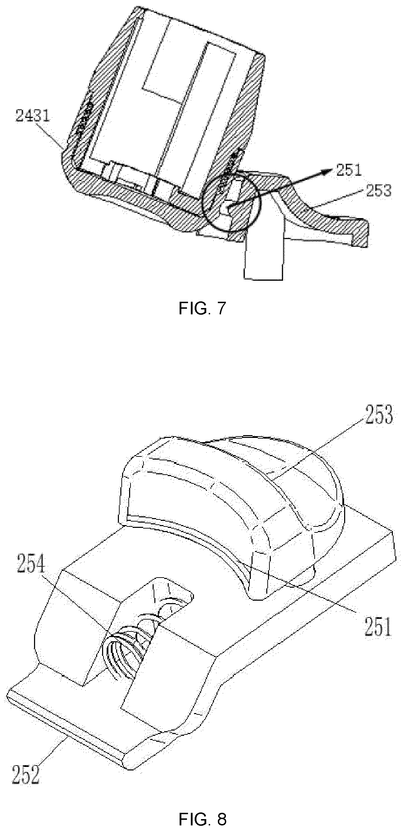

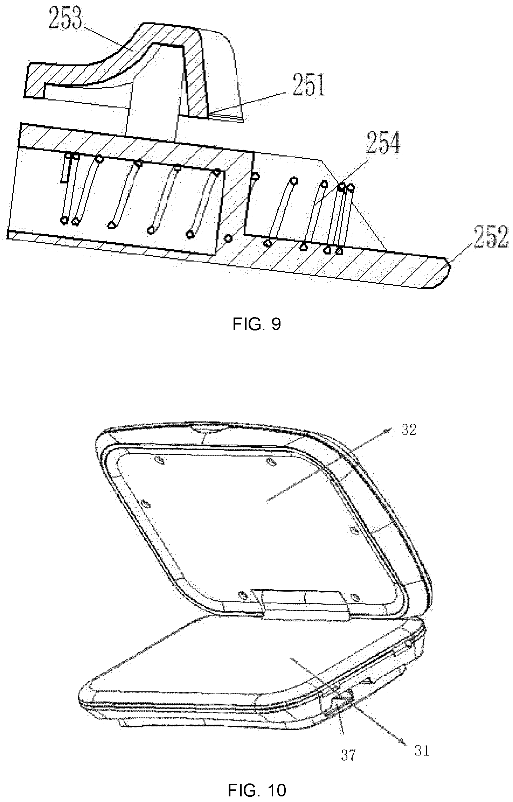

As shown in FIGS. 8 and 9, in some implementations,
the locking key 25 is connected to a slide groove (not shown in drawings) on the support main body 21 in a sliding manner, and an elastic element 254 is provided between the locking key 25 and the support main body 21; and, the elastic element 254 can exert an elastic force to the locking key 25 so that the locking key 25 can slide close to the V6 detection electrode 243 at the retraction position and the embedded electrocardiogram monitor host 3.

In specific use, when it is necessary to use the electrocardiogram monitor for ECG detection, the user exerts a force to the locking key 25 to overcome the elastic force exerted by the elastic element 254, so as to drive the locking key 25 to slide away from the V6 detection electrode 243 and the electrocardiogram monitor host 3. At this time, the first bump 251 escapes from the ring groove 2431 and/or the second bump 252 escapes from the groove, so that the side support arm 24 and/or the electrocardiogram monitor host 3 is unlocked, and the electrocardiogram monitor can be in the ECG detection state. When it is necessary to lock the electrocardiogram monitor host 3 or the side support arm 24, the user only needs to press down the electrocardiogram monitor host 3 or the side support arm 24. When the position of the groove or the ring groove 2431 corresponds to the position of the second bump 252 or the first bump 251, the locking key 25 will slide close to the electrocardiogram monitor host 3 or the side support arm 24 under the elastic force of the elastic element 254, thereby clamping and locking the aforementioned component. Thus, the operation is very simple and convenient. In a preferred embodiment, the locking key 25 has a force exerting bump 253 protruded from the proximal surface of the support main body 21. When the user needs to unlock the side support arm 24 and the electrocardiogram monitor host 3, the user can exert a force to the force exerting bump 253 in the direction away from the aforementioned component, so that the operation is simple and convenient. The aforementioned elastic element 254 can be specifically a spiral spring.

In some implementations,
the insertion fitting depth of the first bump 251 and the ring groove 2431 is less than the insertion fitting depth of the second bump 252 and the groove. As such, by designing the insertion fitting depth of the first bump 251 and the ring groove 2431 to be less than the insertion fitting depth of the second bump 252 and the groove, the electrocardiogram monitor host 3 will not be unlocked at the same time of unlocking the side support arm 24. Specifically, the locking key 25 has two distances for sliding away. The sliding in the shorter distance realizes the unlocking of the side support arm 24, and ECG detection can thus be carried out. The sliding in the longer distance realizes the unlocking of the electrocardiogram monitor host 3, and the host can be replaced in this case. It is to be noted that, when the locking key 25 slides in the above two distances, due to the compression elastic force of the elastic element 254, the long-distance sliding requires the user to exert a greater force, so that the electrocardiogram monitor host 3 can be prevented from being unlocked by mistake when the side support arm 24 is unlocked for ECG detection, thus avoiding damage caused by the accidental fall of the electrocardiogram monitor host 3. As a more preferred implementation, a third bump (not shown in drawings) is provided on an edge of the proximal surface of the side support arm 24; and, when the side support arm 24, the upper support arm 22 and the lower support arm 23 are all at the retraction positions, the third bump is clamped with the V1 detection electrode 221, the V2 detection electrode 222 and the LL detection electrode 231 to prevent the upper support arm 22 and the lower support arm 23 from being turned away from the support main body 21.

In this technical solution, when the electrocardiogram monitor is in the turned and retracted state, the side support arm 24 realizes the mutual locking and limiting of the upper support arm 22 and the lower support arm 23 through the third bump, so that the upper support arm 22 and the lower support arm 23 can be prevented from being turned away from the support main body 21 in the folded and retracted state, and each detection electrode on the upper support arm 22 and the lower support arm 23 can be protected. Thus, it should be understood that, during the retraction of each support arm, the side support arm 24 is turned and retracted after the upper support arm 22 and the lower support arm 23 are turned and retracted in place, so that the side support arm 24 is objectively prevented from being on the turning path of the upper support arm 22 and the lower support arm 23.

The LA detection electrode 211 and the RA detection electrode 212 are detection electrode sheets. When the electrocardiogram monitor is in the ECG detection state, the left and right hands of the user to be detected can be placed on the LA detection electrode 211 and the RA detection electrode 212, respectively, thereby realizing the positioning of the electrocardiogram monitor by the user with hands, and achieving the ECG detection of the user's left and right arms. The V1 detection electrode 221, the V2 detection electrode 222, the V3 detection electrode 213, the V4 detection electrode 241, the V5 detection electrode 242, the V6 detection electrode 243, the RL detection electrode 214 and the LL detection electrode 231 are all detection electrode columns.

As shown in FIGS. 10-13, according to an embodiment of the present invention, specifically referring to FIG. 10, an electrocardiogram monitor host 3 is provided, including a host component 3131 and a display component 3232. The electrocardiogram monitor host 3 is assembled on the distal surface of the support main body 21 through the host component 31. The host component 31 includes a host shell 311 and an ECG detection circuit board 312 arranged in the host shell 311. The ECG detection circuit board 312 is electrically connected to each detection electrode (through a lead, not shown in drawings). The display component 32 includes a display screen assembly 322, which is at least configured to display ECG parameters output by the ECG detection circuit board 312. Specifically, the host shell 311 can include a host top shell and a host bottom shell which are buckled and assembled relative to each other. A host accommodating space is formed between the host top shell and the host bottom shell. The aforementioned ECG detection circuit board 312 is arranged in the host accommodating space, and corresponding electronic components such as a chip are configured on the ECG detection circuit board 312 for electrical connection to each detection electrode assembly, so as to perform conventional operations such as processing and analysis on the acquired ECG signals (bioelectric signals). The operations such as processing and analysis on the aforementioned ECG signals by the ECG detection circuit board 312 are the conventional operations in the art and are not the contents protected by the present invention, and will not be repeated here. The aforementioned ECG parameters are, for example, heart rate, ECG curve or the like. By taking the orientation when using the portable electrocardiogram monitor by the user as reference, the host component 31 is located on the side of the display component 32 close to the detected human body.

In this technical solution, the electrocardiogram monitor host 3 is integrally arranged on the detection electrode support 2, and the display screen assembly 322 of the electrocardiogram monitor host 3 can directly display the ECG parameters output by the ECG detection circuit board 312 without the help of intelligent terminals such as mobile phones and tablet computers. Therefore, the electrocardiogram monitor is not restricted by the terminal equipment or network and is especially suitable for the elderly population, and thus is easy for application and popularization in the elderly population.

In some implementations,
the display component 32 has a display shell 321; the display screen assembly 322 is located on a side face of the display shell 321 away from the host shell 311; a first side of the display shell 321 is pivotally connected to a first side of the host shell 311 through a pivot structure (not marked in the drawings); and, the display component 32 can be driven to unfold or fold around the pivot structure relative to the host component 31. The aforementioned pivot structure can be specifically a common rotating shaft connecting structure. It is preferable to use a commercially-available damping rotating shaft to realize the unfolding or folding connection between the host component 31 and the display component 32, so that it is smooth and stable to unfold or fold the display component 32.

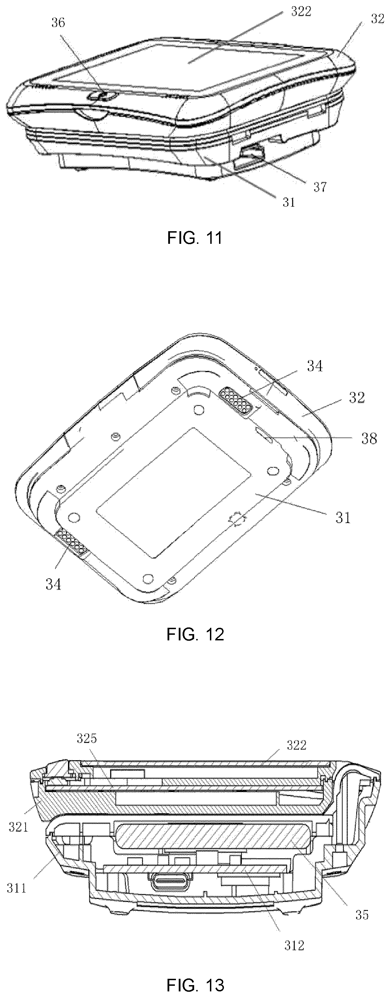

In this technical solution, the display component 32 can be driven (e.g., by manually exerting a force by the user) to unfold (e.g., in the state shown in FIG. 10) or fold (e.g., in the state shown in FIG. 11) around the pivot structure between the display component 32 and the host component 31 relative to the host component 31, and the user can select the unfolding angle according to his or her own viewing angle, so that it is convenient for the user to directly view the ECG parameters on the display screen assembly 322 during ECG detection, and the validity of the operation and detection data can be determined at the first time. When the display component 32 and the host component 31 are in the unfolded state, the maximum unfolding angle formed therebetween is generally designed to be not less than 90 ° , so that the user can reasonably adjust the unfolding angle according to needs. However, it should be understood that, by taking the user's state when using the electrocardiogram monitor as reference, the pivot structure is located on the side close to the user's head, that is, the opening of the unfolding angle faces the side of user's lower limbs.

In some implementations,
the assembling hole 215 is constructed on the support main body 21, and the host component 31 is embedded into the assembling hole 215; an electrical switching component 34 electrically connected to the ECG detection circuit board 312 is provided on a shell bottom wall of the host shell 311, and an electrical connection component 2151 electrically connected to each of the detection electrodes is provided on a hole wall of the assembling hole 215; and, when the host component 31 is assembled in the assembling hole 215, the electrical connection component 2151 is connected to the electrical switching component 34. In this technical solution, the detachable electrical connection between the electrocardiogram monitor host electrocardiogram monitor host 3 and the detection electrode support 2 and each detection electrode thereon is realized by the butt-joint connection between the electrical connection component 2151 on the hole wall of the assembling hole 215 and the electrical switching component 34 on the shell bottom wall of the host shell 311, and the detachable connection between the electrocardiogram monitor host electrocardiogram monitor host 3 and the detection electrode support 2 can improve the maintainability and function expandability of the electrocardiogram monitor.

In a specific embodiment, the electrical connection component 2151 and the electrical switching component 34 are spring pin connectors (e.g., commercially-available pogopin interfaces) which are matched with each other. At this time, the electrical connection component 2151 and the electrical switching component 34 can be electrically butt-jointed in the process of embedding the electrocardiogram monitor host electrocardiogram monitor host 3 in the assembling hole 215. However, it should be understood that the electrocardiogram monitor host electrocardiogram monitor host 3 can be inserted and embedded into the assembling hole 215 in a direction approximately parallel to the depth direction of the assembling hole 215. This assembling mode can make the structural design of the detection electrode support 2 more reasonable, so that it is more convenient to arrange each detection electrode on the detection electrode support 2 at the corresponding detection position. However, it should be noted that, although the aforementioned spring pin connector can facilitate the assembling of the electrocardiogram monitor host electrocardiogram monitor host 3 in the assembling hole 215, the transmission of the ECG parameters (signals) is weakened to a certain extent since the connecting pin in the spring pin connector needs to be elastically expanded or contracted. As a result, it is disadvantage for the subsequent computation and analysis of the ECG signals in the ECG detection circuit board 312, thus reducing the accuracy of the output results. However, the connection between the electrocardiogram monitor host electrocardiogram monitor host 3 and the detection electrode support 2 through the spring pin connector can realize the embedding connection between the both in an integral translation manner, thereby ensuring the reliable electrical connection between the display apparatus and each detection electrode and realizing more compact structure. It can be seen from FIG. 12 that there are two electrical switching components 34, which are located in regions at two ends of the length of the host shell 311, thereby realizing the electrical connection to the detection electrodes in the left and right parts of the detection electrode support.

Specifically referring to FIG. 13, a power supply battery 35 is further provided in the host shell 311, and the power supply battery 35 is electrically connected to the ECG detection circuit board 312 and the display screen assembly 322. The power supply battery 35 can be specifically a rechargeable battery to further improve the portability of the corresponding electrocardiogram monitor. Specifically, a corresponding charging interface 38 is constructed on the host shell 311, and the user charges the aforementioned power supply battery 35 through the charging interface 38. In a preferred embodiment, a display control circuit board 325 is provided in the display shell 321, the power supply battery 35 is also electrically connected to the display control circuit board 325, and a power switch 36 used for controlling the power supply of the power supply battery 35 is provided on the display shell 321. The user can enable or disable the display apparatus by pressing the power switch 36. In a specific embodiment, the aforementioned display control circuit board 325 can be an existing Android control mainboard. The control technology design of the Android control mainboard is relatively mature and enables the display apparatus of the present invention to have more expanded functions. For example, a camera module, a loudspeaker module and a remote control module can be configured on the display control circuit board 325, so that the display apparatus of the present invention can have the function of remote doctor diagnosis and the intelligence of the display apparatus is improved.

According to an embodiment of the present invention, a handheld portable electrocardiogram monitor is provided, including a detection electrode support 2 on which a plurality of detection electrodes is assembled. Each detection electrode is arranged according to the corresponding target ECG detection position. The detection electrode support 2 includes a support main body 21. The handheld portable electrocardiogram monitor further includes the aforementioned electrocardiogram monitor host 3.

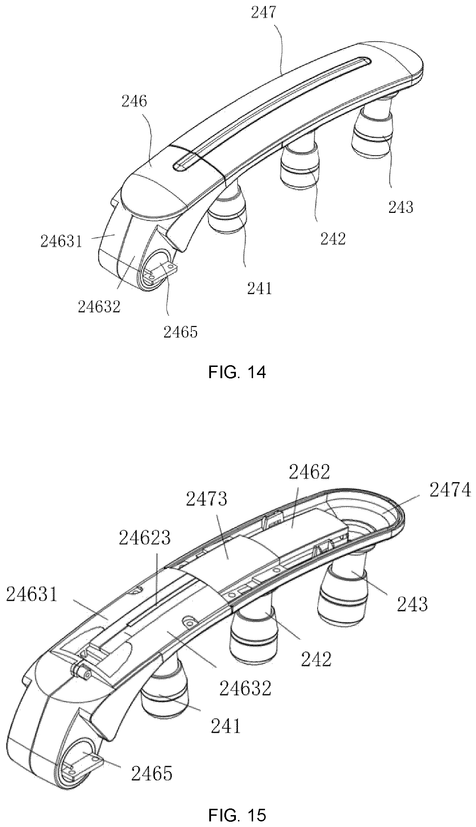

As shown in FIGS. 14-22, according to an embodiment of the present invention, specifically referring to FIG. 14, an ECG detection electrode support arm with adjustable length is provided, including: a fixed arm section 246, a first end of which is used for connection to the support main body 21 of the electrocardiogram monitor in a turnable manner, a first detection electrode 241 being provided on a proximal surface of the fixed arm section 246 (in a specific embodiment, the first detection electrode 241 corresponds to the V4 detection electrode in the 12-leads, and the following description will be given by taking the V4 detection electrode as an example), the fixed arm section 246 being also provided with a slide rail 2462 arranged in a length extension direction thereof, where the aforementioned proximal surface refers to the side of wall on which the corresponding component faces the body of the user to be detected when the electrocardiogram monitor is in the ECG detection state, and the opposite side thereof is the distal surface, i.e., the side of wall on which the corresponding component is away from the body of the user to be detected when the electrocardiogram monitor is in the ECG detection state; and, a telescopic arm section 247, a second detection electrode being provided on a proximal surface of the telescopic arm section 247, the aforementioned second detection electrode being used for positioning the left 3/4 intercostal midaxillary line of the human body of the detected user. With regard to the 12-leads, the second detection electrode at least includes the V6 detection electrode 243. In a preferred embodiment, the second detection electrode further includes the V5 detection electrode 242. A slider 2473 is also provided on the telescopic arm section 247 where the second detection electrode is located. The slider 2473 can be sleeved on the outer side of the slide rail 2462 in a sliding manner in the length direction of the slide rail 2462, so that the telescopic arm section 247 is connected to the second end of the fixed arm section 246 in a slidable and telescopic manner.

In this technical solution, on one hand, the first end of the ECG detection electrode support arm is connected to the support main body 21 in a turnable manner, so that the proximal surface of the support arm can be opposite to the proximal surface of the support main body 21 after being turned, thereby folding and retracting the support arm to improve the structure compactness, at the same time physically protecting each detection electrode on the support arm and avoiding the damage to the detection electrode. On the other hand, on the basis that the support arm can be turned, the support arm is designed as a telescopic structure, so that the second detection electrode (which is the V6 detection electrode 243 in a specific embodiment) on the support arm can be adjusted according to different body shapes of users and accurately positioned at target positions for different body shapes, thereby improving the accuracy of the output results of ECG detection of the electrocardiogram monitor.

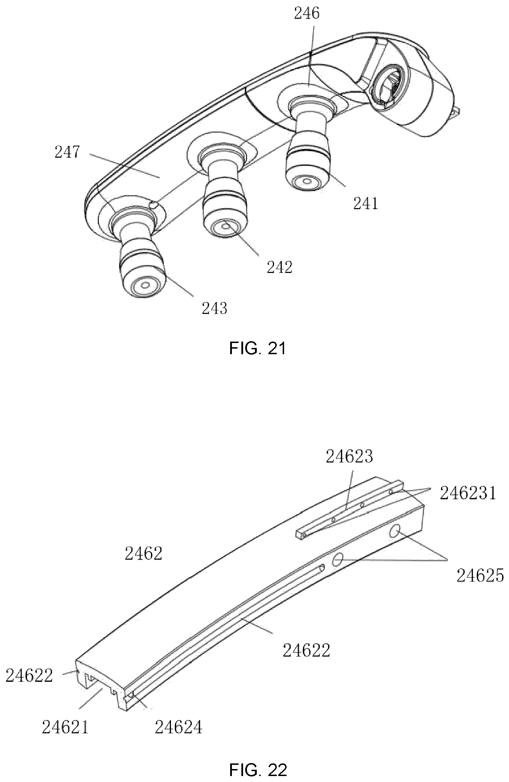

As shown in FIG. 22, in some implementations, the slide rail 2462 has a wiring groove 24621 running through two ends of the slide rail 2462 in its length direction; the wiring groove 24621 has a U-shaped cross-section; the opening of the U-shape faces the proximal surfaces of the fixed arm section 246 and the telescopic arm section 247; and, the respective cables of the first detection electrode 241 and the second detection electrode at least partially pass through the wiring groove 24621. In a specific embodiment, the aforementioned wiring groove 24621 has a width of about 10 mm, and the cable of each detection electrode is distributed in the wiring groove 24621. Since the slide rail 2462 is fixedly connected to the fixed arm section 246, the position of the slide rail 2462 relative to the fixed arm section 246 does not change in the telescopic adjustment process of the telescopic arm section 247, so that the cable is protected, and the adverse wear phenomenon caused by friction on the cable can be avoided during the length adjustment process.

Continuously referring to FIG. 22, each of two opposite sidewalls of the slide rail 2462 is constructed with a slideway 24622 extending in a length direction thereof; the end of the slideway 24622 away from the telescopic arm section 247 is a blind end, while the end of the slideway 24622 close to the telescopic arm section 247 is an open end; a plurality of ball holes (not shown) are formed at positions on the slider 2473 corresponding to each of the slideways 24622, and the plurality of ball holes are arranged at intervals in the length direction of the slider 2473; and, a ball (not shown) is clamped between various ball hole and the slideway 24622.

In this technical solution, by clamping a plurality of balls between the slider 2473 and the slide rail 2462, the relative sliding between the both can be smoother, and the convenience of the length adjustment operation can be improved. It is to be noted that the blind end of the slideway 24622 can limit the maximum retraction position of the slider 2473.

As a more preferred embodiment, a limiting hole 24624 is formed at the open end, and a limiting member (e.g., a limiting pin for interference fit, a limiting stud for threaded connection, etc.) is connected in the limiting hole 24624, thereby limiting the maximum extension position of the telescopic arm section 247 by contacting the side of the slider 2473 away from the fixed arm section 246. Thus, the aforementioned open end realizes the sliding connection between the slider 2473 and the slide rail 2462 by assembling balls, and also limits the maximum extension position of the slider 2473 through the limiting member, so that the structure is simple.

As shown in FIGS. 15 and 22, a positioning rib 24623 is provided on a wall surface of the slide rail 2462 away from the wiring groove 24621, and a plurality of positioning bumps 246231 are provided on the positioning rib 24623. The plurality of positioning bumps 246231 are arranged at intervals in the length direction of the positioning rib 24623. The fixed arm section 246 includes a first fixed shell 24631 and a second fixed shell 24632 which are assembled in the width direction of the fixed arm section 246. A clamping gap (not marked) is formed between the first fixed shell 24631 and the second fixed shell 24632. Positioning grooves (not shown) corresponding to the positioning bumps 246231 are formed on the opposite walls of the clamping gap. The positioning rib 24623 is clamped in the clamping gap, and the positioning bumps 246231 are correspondingly located in the positioning grooves, respectively.

In this technical solution, the fixed arm section 246 is formed by splicing the first fixed shell 24631 and the second fixed shell 24632, so that it is advantageous for the structural design at the first end and it is beneficial for the reduction of the manufacturing difficulty of the fixed arm section 246. At this time, the positioning rib 24623 on the slide rail 2462 can be clamped in the clamping gap formed between the first fixed shell 24631 and the second fixed shell 24632 in the process of assembling the both, and pre-positioning is realized by the positioning bumps 246231 and the positioning grooves, so that the fixed connection between the slide rail 2462 and the fixed arm section 246 is realized, and it is convenient to assemble three separate components and reduce the assembling difficulty.

On the basis of the above pre-positioning, positioning holes 24625 are also constructed on the two sidewalls of the slide rail 2462 on which the slideways 24622 are constructed, and the first fixed shell 24631 and the second fixed shell 24632 are bolted in the positioning holes 24625 through threaded members (e.g., screws).

In other words, on one hand, the slide rail 2462 in the present invention is primarily positioned relative to the first fixed shell 24631 and the second fixed shell 24632 through the positioning rib 24623; on the other hand, the slide rail 2462 is secondarily positioned by the threaded members. Thus, the position reliability of the slide rail 2462 is ensured, and the smoothness of the telescopic adjustment of the telescopic arm section 247 is ensured.

The fixed arm section 246 further includes a first electrode shell 2464, the first detection electrode 241 is located on a proximal surface of the first electrode shell 2464, and the first electrode shell 2464 is detachably connected to the proximal surfaces of the first fixed shell 24631 and the second fixed shell 24632.

In other words, in this technical solution, the first electrode shell 2464 is molded and assembled independently of other components in the fixed arm section 246, so that it can be advantageous for the integral molding of the detection electrodes. At this time, the first fixed shell 24631 and the second fixed shell 24632 are used as one of the connecting components for turning connection on one hand, and are used as the assembling carrier of the first electrode shell 2464 on the other hand.

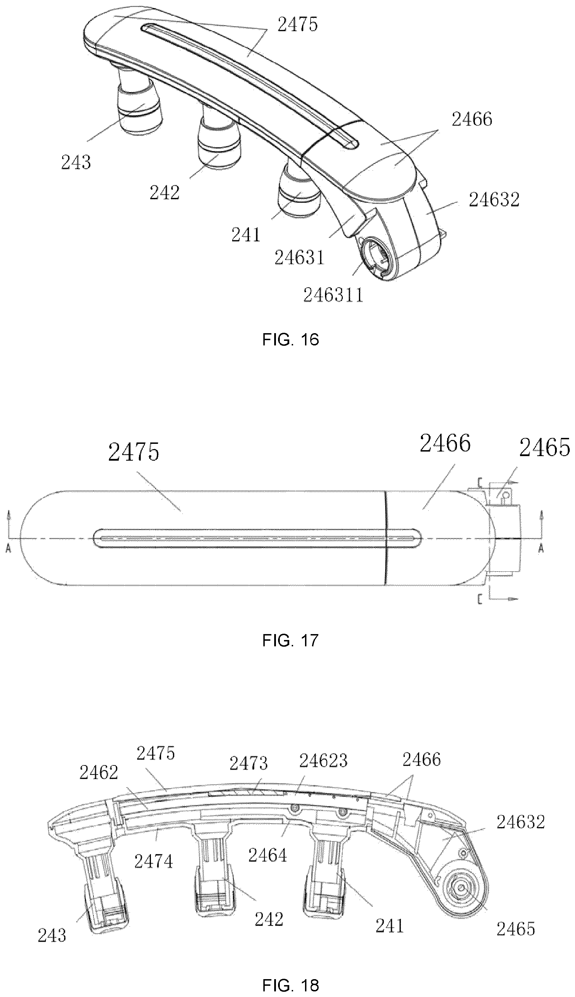

Specifically referring to FIG. 18, the telescopic arm section 247 further includes a second electrode shell 2474, the second detection electrode is located on a proximal surface of the second electrode shell 2474, and the slider 2473 has a U-shaped cross-section. The U-shaped opening of the slider 2473 faces the second electrode shell 2474 and is connected to a distal surface of the second electrode shell 2474. The slider 2473 with the U-shaped section is buckled upside down on three outer side faces of the slide rail 2462, so that the stability and reliability of the sliding connection between the slider 2473 and the slide rail 2462 can be ensured.

In a preferred embodiment, the electrode column casing of the first detection electrode 241 is integrated with the first electrode shell 2464, and the electrode column casing (not marked) of the second detection electrode is integrated with the second electrode shell 2474. No assembling procedure is required for each integrated electrode column casing, so that the assembling efficiency of each detection electrode can be improved.

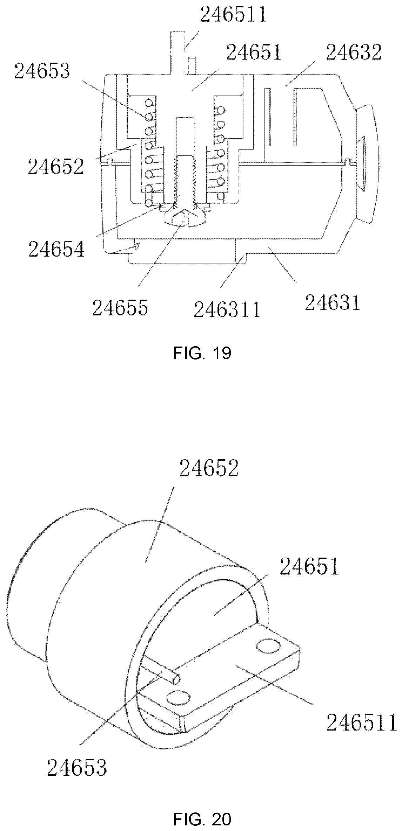

As shown in FIGS. 15 and 19, in some implementations, a first through hole (not marked) is formed at the end of the first fixed shell 24631 corresponding to the first end, and a second through hole (not marked) is formed at the end of the second fixed shell 24632 corresponding to the first end. A flanged ring 246311 is formed at the orifice of the first through hole on the side away from the second fixed shell 24632, and the rotating shaft connection with the corresponding connecting lug on the support main body 21 can be realized through the flanged ring 246311. The respective cables of the first detection electrode 241 and the second detection electrode can be guided out from the hollow structure corresponding to the flanged ring 246311 and the first through hole. Thus, the structural design of the electrocardiogram monitor is simplified.

Specifically referring to FIG. 19, a rotating shaft assembly 2465 is assembled in the second through hole; the rotating shaft assembly 2465 includes a rotating shaft 24651; the rotating shaft 24651 has a connecting portion 246511; the connecting portion 246511 is used for detachable connection to the support main body 21; and, the connecting portion 246511 has an extended connection position protruded from the outer side of the orifice of the second through hole and a retracted insertion position hidden on the inner side of the orifice of the second through hole. In this technical solution, the rotatable connection between the second fixed shell 24632 and the corresponding connecting lug on the support main body 21 is realized through the rotating shaft assembly 2465. With reference to the above text, the first fixed shell 24631 can realize the rotating shaft connection with the corresponding connecting lug on the support main body 21 through the flanged ring 246311. Thus, the first fixed shell and the second fixed shell can realize the turning function of the ECG detection electrode support arm. As shown in FIG. 14, the connecting portion 246511 is specifically of a plate structure on which a connecting hole is formed, the lug connected thereto is correspondingly constructed with a corresponding straight through slot (not shown), and the lug is of a shell structure with an internal space. During assembling the support arm, a force is exerted to the connecting portion 246511 so that the connecting portion 246511 is located at the retracted insertion position. At this time, the first end of the support arm can be inserted into the gap between two lugs, and the flanged ring 246311 is inserted into the lug through hole on one side in a rotatable manner. After the rotating shaft assembly 2465 rotates to be coaxial with the lug through hole, the connecting portion 246511 is switched from the retracted insertion position to the extended connection position, that is, the connecting portion 246511 extends into the through slot. At this time, the position of the connecting portion 246511 can be fixed on the inner side of the support main body 21. In this case, it should be understood that the second fixed shell 24632 can rotate relative to the rotating shaft assembly 2465. In this technical solution, the position switching of the connecting portion 246511 allows the support arm to be assembled independently of the support main body 21 and then allows the first end of the support arm to be integrally assembled with the support main body 21, and it is only necessary to connect the connecting portion 246511 to the support main body 21. The connecting portion 246511 that can be retracted can realize a smaller gap between the assembled support arm and the lug, so that the consistency in appearance is improved.

Further referring to FIG. 19, the rotating shaft assembly 2465 realizes the rotatable connection to the second fixed shell 24632 by the following structure: the rotating shaft assembly 2465 further includes an outer barrel 24652 fixedly connected to the second fixed shell 24632; the rotating shaft 24651 is coaxially connected to the outer barrel 24652 in an axially movable manner; a spiral elastic element 24653 is sleeved on the outer side of the rotating shaft 24651; and, one end of the spiral elastic element 24653 is fixedly connected to the outer barrel 24652, while the other end thereof is fixedly connected to the rotating shaft 24651.

In this technical solution, on one hand, the spiral elastic element 24653 can provide a reverse elastic force to the rotation of the rotating shaft 24651, so that the support arm has a pre-applied force for allowing its proximal surface to approach the proximal surface of the support main body 21, and this force can allow each detection electrode on the support arm to reliably contact the human body in the ECG detection process, thereby ensuring the ECG detection effect. On the other hand, the spiral elastic element 24653 also has a compression resilience force in the axial direction, and the compression resilience force, together with the external force exerted by the user, can drive the axial movement of the connecting portion 246511 (i.e., the rotating shaft 24651), thereby switching between the extended connection position and the retracted insertion position. Specifically, the rotating shaft 24651 has a small-diameter end. The small-diameter end is inserted into the through hole on the bottom of the outer barrel 24652, and the displacement of one side of the small-diameter end is limited by a gasket 24654 and a screw 24655. The rotating shaft 24651 also has a large-diameter end. The spiral elastic element 24653 is clamped between the end face of the large-diameter end and the bottom of the outer barrel 24652. As such, when the user exerts an external force to the connecting portion 246511 to compress the spiral elastic element 24653, the connecting portion 246511 will move close to the bottom of the outer barrel and retract to the second through hole so as to realize the position switching of the connecting portion 246511. On the contrary, after the external force is released, the connecting portion 246511 is extended under the restoring force of the spiral elastic element 24653 so as to switch from the retracted insertion position to the extended connection portion.

As shown in FIG. 14, a fixed section panel 2466 is connected to the distal surface of the fixed arm section 246, and a telescopic section panel 2475 is connected to the distal surface of the telescopic arm section 247, thus improving the overall appearance of the support arm.

The forgoing description merely shows the preferred embodiments of the present invention and is not intended to limit the present invention. Any modification, equivalent replacement and improvement made within the spirit and principle of the present invention shall fall into the protection scope of the present invention. The foregoing description merely shows the preferred implementations of the present invention. It should be pointed out that, to a person of ordinary skill in the art, various improvements and transformations can be made without departing from the technical principle of the present invention and these improvements and transformations shall be regarded as falling into the protection scope of the present invention.

## Claims

1. A portable electrocardiogram monitor, wherein, comprises:
an electrocardiogram detection electrode support (2) and an electrocardiogram monitor host (3);
the electrocardiogram detection support (2) comprises a support main body (21) and support arms;
the support arms comprise an upper support arm (22), a lower support arm (23) and a side support arm (24), and the upper support arm (22), the lower support arm (23) and the side support arm (24) each can be turned by a target angle toward a proximal surface of the support main body (21) around its respective connection with the support main body (21); and
the electrocardiogram monitor host (3) is detachably mounted to the support main body (21).

2. The portable electrocardiogram monitor according to claim 1, wherein,
an LA detection electrode (211) and an RA detection electrode (212) are provided on a distal surface of the support main body (21), and a V3 detection electrode (213) and an RL detection electrode (214) are provided on the proximal surface thereof;
the upper support arm (22) is connected to an upper edge of the support main body (21), and a V1 detection electrode (221) and a V2 detection electrode (222) are provided on a proximal surface of the upper support arm (22);
the lower support arm (23) is connected to a lower edge of the support main body (21), and an LL detection electrode (231) is provided on a proximal surface of the lower support arm (23); and
the side support arm (24) is connected to a left edge of the support main body (21) to correspond to a chest side where the heart of a user to be detected is located, and a V4 detection electrode (241), a V5 detection electrode (242) and a V6 detection electrode (243) are provided on a proximal surface of the side support arm (24).

3. The portable electrocardiogram monitor according to claim 2, wherein,
a locking part is provided on the proximal surface of the support main body (21), and the side support arm (24) has a detection position and a retraction position; when the side support arm (24) is at the detection position, the V4 detection electrode (241), the V5 detection electrode (242) and the V6 detection electrode (243) each is located at a preset electrocardiogram detection position; and, when the side support arm (24) is at the retraction position, the locking part locks the side support arm (24) so that the proximal surface of the side support arm (24) is opposite to the proximal surface of the support main body (21).

4. The portable electrocardiogram monitor according to claim 3, wherein,
the V4 detection electrode (241), the V5 detection electrode (242) and the V6 detection electrode (243) are arranged at intervals in a length direction of the arm body of the side support arm (24) from a side close to the support main body (21) to a side away from the support main body (21); the locking part comprises a locking key (25), the locking key (25) has a first locking position and a first unlocking position; a ring groove (2431) is formed on the V6 detection electrode (243); a first bump (251) is provided on the locking key (25); when the side support arm (24) is at the retraction position, the first bump (251) is at least partially inserted into the ring groove (2431) to be located at the first locking position; and, when the locking key (25) is at the first unlocking position, the first bump (251) escapes from the ring groove (2431).

5. The portable electrocardiogram monitor according to claim 1, wherein,
an assembling hole (215) for allowing the electrocardiogram monitor host (3) to be embedded therein is constructed on the support main body (21); the locking key (25) also has a second locking position and a second unlocking position; a groove is formed on a sidewall of the electrocardiogram monitor host (3); a second bump (252) is also provided on the locking key (25); when the electrocardiogram monitor host (3) is embedded into the assembling hole (215), the second bump (252) is at least partially inserted into the groove to be located at the second locking position; and, when the locking key (25) is at the second unlocking position, the second bump (252) escapes from the groove.

6. The portable electrocardiogram monitor according to claim 5, wherein,
the locking key (25) is connected to a slide groove on the support main body (21) in a sliding manner, and an elastic element (254) is provided between the locking key (25) and the support main body (21); and, the elastic element (254) can exert an elastic force to the locking key (25) so that the locking key (25) can slide close to the V6 detection electrode (243) at the retraction position and the embedded electrocardiogram monitor host (3).

7. The portable electrocardiogram monitor according to claim 6, wherein,
the insertion fitting depth of the first bump (251) and the ring groove (2431) is less than the insertion fitting depth of the second bump (252) and the groove; and/or, the locking key (25) has a force exerting bump (253) protruded from the proximal surface of the support main body (21).

8. The portable electrocardiogram monitor according to claim 2, wherein,
a third bump is provided on an edge of the proximal surface of the side support arm (24); and, when the side support arm (24), the upper support arm (22) and the lower support arm (23) are all at the retraction positions, the third bump is clamped with the V1 detection electrode (221), the V2 detection electrode (222) and the LL detection electrode (231) to prevent the upper support arm (22) and the lower support arm (23) from being turned away from the support main body (21).

9. The portable electrocardiogram monitor according to claim 2, wherein,
the LA detection electrode (211) and the RA detection electrode (212) are detection electrode sheets; and, the V1 detection electrode (221), the V2 detection electrode (222), the V3 detection electrode (213), the V4 detection electrode (241), the V5 detection electrode (242), the V6 detection electrode (243), the RL detection electrode (214) and the LL detection electrode (231) are all detection electrode columns.

10. The portable electrocardiogram monitor according to any one of claims 1 to 9, wherein,
the electrocardiogram monitor host (3) comprises a host component (31) and a display component (32); the electrocardiogram monitor host (3) is assembled on the distal surface of the support main body (21) through the host component (31); the host component (31) comprises a host shell (311) and an electrocardiogram detection circuit board (312) arranged in the host shell (311); the electrocardiogram detection circuit board (312) is electrically connected to each of the detection electrodes; the display component (32) comprises a display screen assembly (322); and, the display screen assembly (322) is at least configured to display electrocardiogram parameters output by the electrocardiogram detection circuit board (312).

11. The portable electrocardiogram monitor according to claim 10, wherein,
the display component (32) has a display shell (321); the display screen assembly (322) is located on a side face of the display shell (321) away from the host shell (311); a first side of the display shell (321) is pivotally connected to a first side of the host shell (311) through a pivot structure; and, the display component (32) can be driven to unfold or fold around the pivot structure relative to the host component (31).

12. The portable electrocardiogram monitor according to claim 10, wherein,
the assembling hole (215) is constructed on the support main body (21), and the host component (31) is embedded into the assembling hole (215); an electrical switching component (34) electrically connected to the electrocardiogram detection circuit board (312) is provided on a bottom wall of the host shell (311), and an electrical connection component (2151) electrically connected to each of the detection electrodes is provided on a wall of the assembling hole (215); and, when the host component (31) is assembled in the assembling hole (215), the electrical connection component (2151) is connected to the electrical switching component (34).

13. The portable electrocardiogram monitor according to any one of claims 1 to 12 wherein,
the length of the side support arm (24) is adjustable, and the side support arm (24) comprises:
a fixed arm section (246), which is connected to the support main body (21) of the electrocardiogram monitor in a turnable manner, the V4 detection electrode (241) being provided on a proximal surface of the fixed arm section (246), the fixed arm section (246) being also provided with a slide rail (2462) arranged in a length extension direction of the fixed arm section (246); and
a telescopic arm section (247), the V5 detection electrode (242) and the V6 detection electrode (243) being provided on a proximal surface of the telescopic arm section (247), a slider (2473) being also provided on the telescopic arm section (247), the slider (2473) being sleeved on the outer side of the slide rail (2462) in the length direction of the slide rail (2462) in a sliding manner.

14. The portable electrocardiogram monitor according to claim 13, wherein,
the slide rail (2462) has a wiring groove (24621) running through two ends of the slide rail (2462) in a length direction thereof; the wiring groove (24621) has a U-shaped cross-section; the opening of the U-shape faces the side of the proximal surfaces of the fixed arm section (246) and the telescopic arm section (247); and, the respective cables of the V4 detection electrode (241), the V5 detection electrode (242) and the V6 detection electrode (243) at least partially pass through the wiring groove (24621).

15. The portable electrocardiogram monitor according to claim 14, wherein,
each of two opposite sidewalls of the slide rail (2462) is constructed with a slideway (24622) extending in a length direction thereof, a plurality of ball holes are formed at positions on the slider (2473) corresponding to each of the slideways (24622), and a ball is clamped between each of the ball holes and the slideway (24622).

16. The portable electrocardiogram monitor according to claim 15, wherein,
a positioning rib (24623) is provided on a wall surface of the slide rail (2462) away from the wiring groove (24621), and a plurality of positioning bumps (246231) are provided on the positioning rib (24623).

17. The portable electrocardiogram monitor according to claim 16, wherein,
a limiting hole (24624) is formed at an open end of the slideway (2462), and a limiting member is connected in the limiting hole (24624); and/or, positioning holes (24625) are also constructed on the two sidewalls of the slide rail (2462) on which the slideways (24622) are constructed.

18. The portable electrocardiogram monitor according to claim 16, wherein,
the fixed arm section (246) further comprises a first electrode shell (2464), and the V4 detection electrode (241) is located on a proximal surface of the first electrode shell (2464).

19. The portable electrocardiogram monitor according to claim 18, wherein,
the telescopic arm section (247) further comprises a second electrode shell (2474), and the V5 detection electrode (242) and the V6 detection electrode (243) are located on a proximal surface of the second electrode shell (2474); and/or, the electrode column casing of the V4 detection electrode (241) is integrated with the first electrode shell (2464), and the electrode column casings of the V5 detection electrode (242) and the V6 detection electrode (243) are integrated with the second electrode shell (2474).

20. The portable electrocardiogram monitor according to claim 16, wherein,
a rotating shaft assembly (2465) is assembled on the fixed arm section (246); and the rotating shaft assembly (2465) comprises a rotating shaft (24651); the rotating shaft (24651) has a connecting portion (246511); and, the connecting portion (246511) is used for detachable connection to the support main body (21).

21. The portable electrocardiogram monitor according to claim 20, wherein,
the rotating shaft assembly (2465) further comprises an outer barrel (24652); the rotating shaft (24651) is coaxially connected to the outer barrel (24652) in an axially movable manner; a spiral elastic element (24653) is sleeved on the outer side of the rotating shaft (24651); and, one end of the spiral elastic element (24653) is fixedly connected to the outer barrel (24652), while the other end thereof is fixedly connected to the rotating shaft (24651).
